# EUROPEAN PATENT SPECIFICATION

(11) **EP 1 666 043 B1**
(45) Date of publication and mention of the grant of the patent: **02.12.2009**
(21) Application number: 04771296.3
(22) Date of filing: 30.07.2004
(51) Int. Cl.: A61K 31/5575, A61K 9/00, A61K 47/26, B65D 1/02

(54) **PRODUCT CONTAINING PROSTAGLANDIN**
PROSTAGLANDIN ENTHALTENDES PRODUKT
PRODUIT CONTENANT DE LA PROSTAGLANDINE

(30) Priority: 31.07.2003 JP 2003283840
(43) Date of publication of application: 07.06.2006
(73) Proprietor: SANTEN PHARMACEUTICAL CO., LTD., Higashiyodogawa-ku, Osaka-shi, Osaka 533-8651 (JP)
(72) Inventor: KIMURA, Akio, c/o SANTEN PHARMACEUTICAL CO., LTD., Osaka-shi, Osaka 5338651 (JP); YAMADA, Hiroshi c/o SANTEN PHARMACEUTICAL CO., LTD, Osaka-shi, Osaka 5338651 (JP); KADO, Takehiro c/o SANTEN PHARMACEUTICAL CO., LTD., Osaka-shi, Osaka 5338651 (JP); IKEDA, Masayuki c/o SANTEN PHARMACEUTICAL CO., LTD, Osaka-shi, Osaka 5338651 (JP)
(74) Representative: Peaucelle, Chantal
(86) International application number: PCT/JP2004/011282
(87) International publication number: WO 2005/011704

(56) References cited:
- EP-A- 1 321 144
- WO-A-02/22106
- JP-A- 7 033 650
- JP-A- 57 131 242
- JP-A- 62 194 861
- JP-A- 2002 161 037
- JP-A- 2002 520 368

## Description

### TECHNICAL FIELD

The present invention relates to a product which contains prostaglandin, wherein an aqueous liquid preparation containing a prostaglandin derivative that is liable to be adsorbed on a container and slightly soluble in water is stored in a resin container formed from a polymer alloy of polyethylene terephthalate and polyarylate, thereby inhibiting the decrease of the content of the prostaglandin derivative in the aqueous liquid preparation.

### BACKGROUND ART

Prostaglandin is a physiologically active substance, and a number of prostaglandin derivatives have been studied and developed. For ophthalmic applications, for example, prostaglandin derivatives which are useful as therapeutic agents for glaucoma and ocular hypertension are reported in Japanese Patent No. 2721414, and JP-A Nos. H02-108 and H11-71344.

However, some prostaglandin derivatives are liable to be adsorbed on a container and have a property being slightly soluble in water. For aqueous liquid preparations containing the prostaglandin derivative having such properties, it is necessary to improve the matters of adsorptivity on a container and solubility in water. On the other hand, known materials of resin containers for storing an aqueous liquid preparation such as eye drops are exemplified by polyethylene, polypropylene, polyethylene terephthalate, polyvinyl chloride, acrylic resins, polystyrene, polymethylmethacrylate, nylon 6.

JP-T No. 2002-520368 (the term "JP-T" as used herein means a published Japanese translation of a PCT application) discloses that a prostaglandin product containing prostaglandin and a surfactant is more stabilized in the case in which it is stored in a polypropylene resin container than in the case in which it is stored in a polyethylene resin container. Also, WO 2002/22106 A1 discloses that prostaglandin can be stably stored in a resin container comprising polypropylene. JP-A No. 2002-161037 discloses an invention wherein solubility in water and adsorptivity on a resin container of a prostaglandin derivative are improved by incorporating a nonionic surfactant such as polysorbate 80 or polyoxyethylene hydrogenated castor oil 60 in an aqueous liquid preparation. However, the resin container itself for storing an aqueous liquid preparation has not been studied.

### DISCLOSURE OF THE INVENTION

In an attempt to store in a stable manner an aqueous liquid preparation containing a prostaglandin derivative that is liable to be adsorbed on a container and slightly soluble in water, it is an important matter to inhibit the decrease of the content of the prostaglandin derivative that is an active ingredient in the aqueous liquid preparation taking into account of the material of a resin container.

Hence, the present inventors elaborately made studies of materials of resin containers suited for storing an aqueous liquid preparation containing a prostaglandin derivative that is liable to be adsorbed on a container and slightly soluble in water, and found that the decrease of the content of the prostaglandin derivative in an aqueous liquid preparation can be markedly inhibited when stored in a resin container formed from a polymer alloy of polyethylene terephthalate and polyarylate. Thus, the present invention was accomplished.

More specifically, the invention relates to the following aspects.
(1) A prostaglandin-containing product, wherein an aqueous liquid preparation containing a prostaglandin derivative that is liable to be adsorbed on a container and slightly soluble in water is stored in a resin container formed from a polymer alloy of polyethylene terephthalate and polyarylate, thereby inhibiting the decrease of the content of the prostaglandin derivative in the aqueous liquid preparation.
(2) The prostaglandin-containing product according to the above aspect (1) wherein the prostaglandin derivative that is liable to be adsorbed on the container and slightly soluble in water is a prostaglandin F2α derivative having a fluorine atom or fluorine atoms in the molecule or a salt thereof.
(3) The prostaglandin-containing product according to the above aspect (2) wherein the prostaglandin F2α derivative having a fluorine atom or fluorine atoms in the molecule is a difluoroprostaglandin F2α derivative.
(4) The prostaglandin-containing product according to the above aspect (1) wherein ratio of components polyethylene terephthalate to polyarylate in the polymer alloy is polyethylene terephthalate/polyarylate = 1/4 to 4/1.
(5) The prostaglandin-containing product according to the above aspect (1) wherein a nonionic surfactant is comprised in the aqueous liquid preparation.
(6) The prostaglandin-containing product according to the above aspect (5) wherein the nonionic surfactant is polysorbate 80 or polyoxyethylene hydrogenated castor oil 60.
(7) The prostaglandin-containing product according to any one of the above aspects (1) to (6) wherein the aqueous liquid preparation is an eye drop.
(8) A method of inhibiting the decrease of the content of a prostaglandin derivative in an aqueous liquid preparation, comprising storing the aqueous liquid preparation containing a prostaglandin derivative that is liable to be adsorbed on a container and slightly soluble in water, in a resin container formed from a polymer alloy of polyethylene terephthalate and polyarylate.
(9) A resin container formed from a polymer alloy of polyethylene terephthalate and polyarylate for storing an aqueous liquid preparation containing a prostaglandin derivative that is liable to be adsorbed on a container and slightly soluble in water.

The prostaglandin derivative used in the invention is not particularly limited as long as it is a prostaglandin derivative that is liable to be adsorbed on a container and slightly soluble in water (hereinafter, referred to as "the present prostaglandin derivative") but can be preferably a prostaglandin F2α derivative having a fluorine atom or fluorine atoms in the molecule disclosed in JP-A No. H11-71344 or H10-251225, more preferably, a difluoroprostaglandin F2α derivative disclosed in JP-A No. H11-71344, and particularly preferably a difluoroprostaglandin F2α derivative having two fluorine atoms at position 15 disclosed in JP-A No. H11-71344. Specific examples of particularly preferred prostaglandin derivative are 16-phenoxy-15-deoxy-15, 15-difluoro-17, 18, 19, 20-tetranorprostaglandin F2α, 16-(3-chlorophenoxy)-15-deoxy-15, 15-difluoro-17, 18, 19, 20-tetranorprostaglandin F2α, 16-phenoxy-15-deoxy-15, 15-difluoro-13, 14-dihydro-17, 18, 19, 20-tetranorprostaglandin F2α, or alkyl esters thereof, or salts of the same. Specific examples of the alkyl ester are lower alkyl esters such as methyl ester, ethyl ester, propyl ester, isopropyl ester, tert-butyl ester, pentyl ester and hexyl ester.

The "prostaglandin derivative being liable to be adsorbed on a container" referred to herein means that the content (the "content" referred to herein meaning the amount that is present as being dissolved in an aqueous solution to the amount of the present prostaglandin allowed for dissolution) of the prostaglandin derivative is drastically reduced when a prostaglandin aqueous solution is stored in a container. For example, when the concentration of the aqueous solution of the present prostaglandin derivative is 0.001% ("%" indicating "% by weight" unless otherwise described especially, same in the followings), the phrase refers to the state in which the compound is adsorbed on the container in an amount of 10% or more after storing in a polyethylene container or a polypropylene container at 60°C for one week.

Also, the "prostaglandin derivative that is slightly soluble in water" referred to herein means one which requires 1000 ml or more water for dissolving 1 g of the prostaglandin derivative (The Pharmacopeia of Japan, thirteenth ed., Description, General principle A-51 (1996)).

In the invention, the "resin container formed from a polymer alloy of polyethylene terephthalate and polyarylate" means a resin container made from a polymer alloy obtained by polymer-alloying polyethylene terephthalate with polyarylate. Examples of the process for the polymer alloying are block copolymerization, graft copolymerization, polymer blend . The polyethylene terephthalate is a polycondensate of ethylene glycol and terephthalic acid or terephthalate diester, while the polyarylate is a polycondensate of bisphenol A and terephthalic acid, isophthalic acid or an ester thereof,

. The polyethylene terephthalate and polyarylate used in the invention can be obtained by any polycondensation method.

The polymer alloy of polyethylene terephthalate and polyarylate used in the invention can be obtained by any process of block copolymerization, graft copolymerization or polymer blend, and can be obtained by, for example, adding an additive such as an alkali metal salt or a heat stabilizer to a mixture of the polyethylene terephthalate and the polyarylate, followed by heating. Examples of commercially available polymer alloy of polyethylene terephthalate and polyarylate are U-8000, U-8400H manufactured by Unitika Ltd., .

Ratio of components in the polymer alloy of polyethylene terephthalate to polyarylate is not particularly limited, but can be preferably polyethylene terephthalate/polyarylate = 1/4 to 4/1, more preferably polyethylene terephthalate/polyarylate = 1/3 to 3/1, and still more preferably polyethylene terephthalate/polyarylate = 1/2 to 2/1.

The resin container formed from a polymer alloy of polyethylene terephthalate and polyarylate is obtained by fabrication of the polymer alloy of polyethylene terephthalate and the polyarylate. Examples of the process for the fabrication are injection blow molding. The shape of the container is not any how limited.

According to the invention, a nonionic surfactant can be added to the aqueous liquid preparation, and thus, the nonionic surfactant inhibits the decrease of the content of the present prostaglandin derivative in the aqueous liquid preparation through improving the solubility in water of the present prostaglandin derivative. Specific examples of the nonionic surfactant are polyoxyethylene fatty acid esters such as polysorbate 80 [polyoxyethylene sorbitan monooleate], polysorbate 60 [polyoxyethylene sorbitan monostearate], polysorbate 40 [polyoxyethylene sorbitan monopalmitate], polyoxyethylene sorbitan monolaurate, polyoxyethylene sorbitan trioleate and polysorbate 65 [polyoxyethylene sorbitan tristearate]; polyoxyethylene hydrogenated castor oils such as polyoxyethylene hydrogenated castor oil 10, polyoxyethylene hydrogenated castor oil 40, polyoxyethylene hydrogenated castor oil 50 and polyoxyethylene hydrogenated castor oil 60; polyoxyethylene polyoxypropylene glycols such as polyoxyethylene (160) polyoxypropylene (30) glycol [Pluronic F68], polyoxyethylene (42) polyoxypropylene (67) glycol [Pluronic P123], polyoxyethylene (54) polyoxypropylene (39) glycol [Pluronic P85], polyoxyethylene (196) polyoxypropylene (67) glycol [Pluronic F127] and polyoxyethylene (20) polyoxypropylene (20) glycol [Pluronic L-44] ; polyoxyl 40 stearate, sucrose fatty acid esters and the like. Preferred examples of the nonionic surfactant are polysorbate 80 [polyoxyethylene sorbitan monooleate], polyoxyethylene hydrogenated castor oil 60, polyoxyl 40 stearate . These nonionic surfactants can be used alone, or in combination of two or more thereof. Particularly preferred nonionic surfactant is polysorbate 80 [polyoxyethylene sorbitan monooleate] or polyoxyethylene hydrogenated castor oil 60, which has been widely used as an additive of eye drops.

It is desired that the prostaglandin-containing product of the invention exists in the state in which the present prostaglandin is dissolved in water, while the amount (concentration) of the present prostaglandin can be selected appropriately in consideration of the application and the like of the aqueous liquid preparation. For example, when eye drops are prepared, the amount (concentration) of the present prostaglandin derivative in the eye drops can be selected appropriately depending on the objective disease, symptoms and the like, which can be preferably 0.00005 to 0.05%. Moreover, when the nonionic surfactant is added to an eye drop, the amount of the nonionic surfactant can be also altered appropriately depending on the amount of the present prostaglandin derivative. However, in light of improvement of the solubility in water of the present prostaglandin derivative, the concentration of the nonionic surfactant is preferably selected to be five times or greater the concentration of the present prostaglandin derivative. Moreover, when the solubility in water must be further elevated, the concentration is particularly preferably selected to be 10 times or greater.

When the prostaglandin-containing product of the invention is an eye drop, a variety of pharmaceutically acceptable additives, e.g., an anti-oxidant such as ethylenediamine tetraacetic acid or dibutyl hydroxytoluene; a tonicity agent such as sodium chloride, potassium chloride, calcium chloride, glycerol or propylene glycol; a buffer such as boric acid, borax, citric acid, disodium hydrogenphosphate or ε-aminocaproic acid; a preservative such as benzalkonium chloride, chlorhexidine gluconate, benzethonium chloride, sorbic acid, potassium sorbate, ethyl parahydroxybenzoate or butyl parahydroxybenzoate, can be added in addition to the aforementioned nonionic surfactant. The method for preparing the eye drop containing the present prostaglandin derivative can be any conventional method for preparation without need of special procedure or operation. Also, it is preferred that the pH of the eye drop be adjusted to 3 to 8, and in particular, 4 to 7.

When the aqueous liquid preparation of the invention is stored in the resin container formed from a polymer alloy of polyethylene terephthalate and polyarylate, although will be explained in detail in the section of storage stability test described later, the decrease of the content of the present prostaglandin derivative in the aqueous liquid preparation can be markedly inhibited in comparison with the cases in which it is stored in any one of polyethylene containers, polypropylene containers and polyethylene terephthalate containers.

### BEST MODE FOR CARRYING OUT THE INVENTION

Hereinafter, the present invention will be described in detail through carrying out storage stability tests (60°C, for one week), however, such descriptions are disclosed for the purpose of better understandings of the invention

### [Storage Stability Test]

### (1) Preparation of Eye Drop

As a typical example of the present prostaglandin derivative, 0.0015% 16-phenoxy-15-deoxy-15, 15-difluoro-17, 18, 19, 20-tetranorprostaglandin F2α isopropyl ester (hereinafter, referred to as the present compound) was used. The present compound was dissolved in purified water using a nonionic surfactant (polysorbate 80), and thereafter, an osinoregulating agent or the like used usually in eye drops was added thereto to give an eye drop having the osmotic pressure of about 1, and the pH of about 6.

### (2) Manufacture of Resin Container

The resin container was obtained through fabrication by injection blow molding of a polymer alloy of polyethylene terephthalate and polyarylate [U-8000 (manufactured by Unitika Ltd.), with the polyethylene terephthalate of about 45%, and the polyarylate of about 55%]. Also, the resin container for comparison was obtained through fabrication by injection blow molding of polyethylene [Petrocene 175K (manufactured by Tosoh Corporation), low density polyethylene], polypropylene [J-225W (manufactured by Mitsui Chemicals, Inc.)] and polyethylene terephthalate [PIFG5H (manufactured by Kanebo Gohsen, Ltd.)], respectively. All of the containers are containers for eye drops having the same shape.

### (3) Test Method

After subjecting each resin container obtained in the above section "(2) Manufacture of Resin Container" to a sterilization treatment, therein was charged the eye drop obtained in the above section " (1) Preparation of Eye Drops". Then, these samples were placed in an aluminum moisture-proof bag. After storage at 60°C for one week, the content of the present compound in each resin container was measured by a high performance liquid chromatographic method. The obtained results are shown in Table 1. In Example and Comparative Examples in the Table, each content value is the mean value of three cases. Moreover, the content of the present compound was determined using as the standard (100%), the content of the present compound following storage at 5°C for one week of the eye drop obtained in the above section "(1) Preparation of Eye Drop" charged in a glass container followed by sealing.

**Table 1**

| | Material of the container | Content of the present compound (%) |
|---|---|---|
| Example 1 | PET /PAR*¹ | 97.0 |
| Comparative Example 1 | LDPE*² | 83.1 |
| Comparative Example 2 | PP*³ | 91.0 |
| Comparative Example 3 | PET*⁴ | 91.8 |

| | | |
|---|---|---|
| *1: U-8000 (manufactured by Unitika Ltd.) *2: Petrocene 175K (manufactured by Tosoh Corporation) *3: J-225W (manufactured by Mitsui Chemicals, Inc.) *4: PIFG5H (manufactured by Kanebo Gohsen, Ltd.) | | |

### (4) Conclusion

As is clear from Table 1, when the present compound was stored in a resin container formed from a polymer alloy of polyethylene terephthalate and polyarylate, excellent storage stability was achieved exhibiting higher content of the present compound in comparison with the cases in which it was stored in any of a polyethylene container, a polypropylene container and a polyethylene terephthalate container.

### INDUSTRIAL APPLICABILITY

According to the present invention, the decrease of the content of the prostaglandin derivative that is an active ingredient in an aqueous liquid preparation is inhibited, thereby enabling storage in a stable manner of an aqueous liquid preparation containing a prostaglandin derivative that is liable to be adsorbed on a container and slightly soluble in water.

## Claims

1. A prostaglandin-containing product, wherein an aqueous liquid preparation containing a prostaglandin F2α derivative having a fluorine atom or fluorine atoms in a molecule is stored in a resin container formed from a polymer alloy of polyethylene terephthalate and polyarylate of which component ratio is polyethylene terephthalate/polyarylate = 1/2 to 2/1, thereby inhibiting the decrease of the content of the prostaglandin F2α derivative in the aqueous liquid preparation.

2. The prostaglandin-containing product according to claim 1 wherein the prostaglandin F2α derivative is a difluoroprostaglandin F2α derivative.

3. The prostaglandin-containing product according to claim 1 wherein the prostaglandin F2α derivative is 16-phenoxy-15-deoxy-15, 15-difluoro-17, 18, 19, 20-tetranorprostaglandin F2α, 16-(3-chlorophenoxy)-15-deoxy-15, 15-difluoro-17, 18, 19, 20-tetranorprostaglandin F2α, 16-phenoxy-15-deoxy-15, 15-difluoro-13, 14-dihydro-17, 18, 19, 20-tetranorprostaglandin F2α, or alkyl ester thereof, or salts of thereof.

4. A method of inhibiting the decrease of the content of a prostaglandin F2α derivative having a fluorine atom or fluorine atoms in a molecule in an aqueous liquid preparation, comprising storing the aqueous liquid preparation containing the prostaglandin F2α derivative in a resin container formed from a polymer alloy of polyethylene terephthalate and polyarylate of which component ratio is polyethylene terephthalate/polyarylate = 1/2 to 2/1.

5. The method according to claim 4 wherein the prostaglandin F2α derivative is a difluoroprostaglandin F2α derivative.

6. The method according to claim 4 wherein the prostaglandin F2α derivative is 16-phenoxy-15-deoxy-15, 15-difluoro-17, 18, 19, 20-tetranorprostaglandin F2α, 16-(3-chlorophenoxy)-15-deoxy-15, 15-difluoro-17, 18, 19, 20-tetranorprostaglandin F2α, 16-phenoxy-15-deoxy-15, 15-difluoro-13, 14-dihydro-17, 18, 19, 20-tetranorprostaglandin F2α, or alkyl ester thereof, or salts of the same.

## Patentansprüche

1. Prostaglandin enthaltendes Erzeugnis, wobei ein wässriges flüssiges Präparat, das ein Prostaglandin-F_{2α}-Derivat mit einem oder mehreren Fluoratomen in dem Molekül enthält, in einem Harzbehältnis, das aus einer Polymerlegierung aus Polyethylenterephthalat und Polyarylat gebildet ist, deren Komponentenverhältnis von Polyethylenterephthalat/Polyarylat = 1/2 bis 2/1 beträgt, wodurch die Abnahme des Gehalts an dem Prostaglandin-F_{2α}-Derivat in dem wässrigen flüssigen Präparat gehemmt wird, aufbewahrt wird.

2. Prostaglandin enthaltendes Erzeugnis nach Anspruch 1, wobei das Prostaglandin-F_{2α}-Derivat ein Difluorprostaglandin-F_{2α}-Derivat ist.

3. Prostaglandin enthaltendes Erzeugnis nach Anspruch 1, wobei das Prostaglandin-F_{2α}-Derivat 16-Phenoxy-15-deoxy-15,15-difluor-17,18,19,20-tetranorprostaglandin F_{2α}, 16-(3-Chlorphenoxy)-15-deoxy-15,15-difluor-17,18,19,20-tetranorprostaglandin F_{2α}, 16-Phenoxy-15-deoxy-15,15-difluor-13,14-dihydro-17,18,19,20-tetranorprostaglandin F_{2α} oder ein Alkylester bzw. Salz davon ist.

4. Verfahren zum Hemmen der Abnahme des Gehalts an einem Prostaglandin-F_{2α}-Derivat mit einem oder mehreren Fluoratomen im Molekül in einem wässrigen flüssigen Präparat, welches das Aufbewahren des wässrigen flüssigen Präparats, das das Prostaglandin-F_{2α}-Derivat enthält, in einem Harzbehältnis, das aus einer Polymerlegierung aus Polyethylenterephthalat und Polyarylat gebildet ist, deren Komponentenverhältnis von Polyethylenterephthalat/Polyarylat = 1/2 bis 2/1 beträgt, umfasst.

5. Verfahren nach Anspruch 4, wobei das Prostaglandin-F_{2α}-Derivat ein Difluorprostaglandin-F_{2α}-Derivat ist.

6. Verfahren nach Anspruch 4, wobei das Prostaglandin-F_{2α}-Derivat 16-Phenoxy-15-deoxy-15,15-difluor-17,18,19,20-tetranorprostaglandin F_{2α}, 16-(3-Chlorphenoxy)-15-deoxy-15,15-difluor-17,18,19,20-tetranorprostaglandin F_{2α}, 16-Phenoxy-15-deoxy-15,15-difluor-13,14-dihydro-17,18,19,20-tetranorprostaglandin F_{2α} oder ein Alkylester bzw. Salz davon ist.

## Revendications

1. Produit contenant de la prostaglandine, dans lequel une préparation liquide aqueuse contenant un dérivé de prostaglandine F2α comprenant un ou plusieurs atomes de fluor dans une molécule est entreposée dans un récipient de résine formé à partir d'un alliage de polymères poly(téréphtalate d'éthylène) et polyarylate dont le rapport des composants poly(téréphtalate d'éthylène)/polyarylate est de 1/2 à 2/1, inhibant ainsi la réduction de la teneur en dérivé de prostaglandine F2α dans la préparation liquide aqueuse.

2. Produit contenant de la prostaglandine selon la revendication 1, dans lequel le dérivé de prostaglandine F2α est un dérivé de difluoroprostaglandine F2α.

3. Produit contenant de la prostaglandine selon la revendication 1, dans lequel le dérivé de prostaglandine F2α est la 16-phénoxy-15-désoxy-15,15-difluoro-17,18,19,20-tétranorprostaglandine F2α, la 16-(3-chlorophénoxy)-15-désoxy-15,15-difluoro-17,18,19,20-tétranorprostaglandine F2α, la 16-phénoxy-15-désoxy-15,15-difluoro-13,14-dihydro-17,18,19,20-tétranorprostaglandine F2α, ou un ester alkylique de celles-ci, ou des sels de celles-ci.

4. Procédé d'inhibition de la réduction de la teneur en un dérivé de prostaglandine F2α, comprenant un ou plusieurs atomes de fluor dans une molécule, dans une préparation liquide aqueuse, consistant à entreposer la préparation liquide aqueuse contenant le dérivé de prostaglandine F2α dans un récipient de résine formé à partir d'un alliage de polymères poly(téréphtalate d'éthylène) et polyarylate dont le rapport des composants poly(téréphtalate d'éthylène)/polyarylate est de 1/2 à 2/1.

5. Procédé selon la revendication 4, dans lequel le dérivé de prostaglandine F2α est un dérivé de difluoroprostaglandine F2α.

6. Procédé selon la revendication 4, dans lequel le dérivé de prostaglandine F2α est la 16-phénoxy-15-désoxy-15,15-difluoro-17,18,19,20-tétranorprostaglandine F2α, la 16-(3-chlorophénoxy)-15-désoxy-15,15-difluoro-17,18,19,20-tétranorprostaglandine F2α, la 16-phénoxy-15-désoxy-15,15-difluoro-13,14-dihydro-17,18,19,20-tétranorprostaglandine F2α, ou un ester alkylique de celles-ci, ou des sels de celles-ci.
